Europäisches Patentamt

European Patent Office

Office européen des brevets

(11) Veröffentlichungsnummer : **0 076 367**
**B1**

(12)

# EUROPÄISCHE PATENTSCHRIFT

(45) Veröffentlichungstag der Patentschrift :
09.10.85

(21) Anmeldenummer : 82106636.2

(22) Anmeldetag : 23.07.82

(51) Int. Cl.⁴ : **B 01 J 19/02, C 07 C 51/14**

(54) **Verwendung von Metallvorrichtungen bei der Umsetzung und Aufarbeitung von Fluorwasserstoff und organische Carbonsäuren oder Kohlenmonoxid enthaltenden Gemischen.**

(30) Priorität : 06.10.81 DE 3139653

(43) Veröffentlichungstag der Anmeldung :
13.04.83 Patentblatt 83/15

(45) Bekanntmachung des Hinweises auf die Patenterteilung : 09.10.85 Patentblatt 85/41

(84) Benannte Vertragsstaaten :
DE FR GB IT NL

(56) Entgegenhaltungen :
US-A- 2 768 983
US-A- 3 116 978
US-A- 3 993 475

(73) Patentinhaber : Röhm GmbH
Kirschenallee Postfach 4242
D-6100 Darmstadt 1 (DE)

(72) Erfinder : Neumann, Alfred
Hermann-Hesse-Strasse 9
D-6096 Raunheim (DE)
Erfinder : Plösser, Willi
Eberstädter Strasse 23
D-6104 Seeheim-Jugenheim 3 (DE)
Erfinder : Siegert, Hermann-Josef, Dr., Dipl.-Chem.
Inselstrasse 21
D-6100 Darmstadt (DE)

## Beschreibung

### Technisches Gebiet

Die Erfindung betrifft die Umsetzung oder Aufarbeitung von Gemischen, die wesentliche Mengen an Fluorwasserstoff neben organischen Carbonsäuren und/oder Wasser und/oder Kohlenmonoxid enthalten, unter Verwendung von korrosionsbeständigen Vorrichtungen aus Metall als Umsetzungs- oder Aufarbeitungsgefäß. Gemische der genannten Art treten bei der Koch'schen Carbonsäuresynthese auf, wenn Fluorwasserstoff als Koch'scher Katalysator eingesetzt wird.

### Stand der Technik

Es versteht sich von selbst, daß beim Arbeiten mit Fluorwasserstoff Werkstoffe verwendet werden müssen, die dagegen beständig sind. Das gilt schon für Ansätze im Laboratoriumsmaßstab, für die Glasgefäße ungeeignet sind. In der US-PS 3 910 963 werden Vorrichtungen aus Polymeren von Fluorchlorkohlenwasserstoffen genannt. Takezaki et al. (Bulletin of the Japan Petroleum Institute, Vol. 8, 1966, S. 31-38, bes. S. 32) verwendete für systematische Untersuchungen einen Autoklaven aus nichtrostendem Stahl.

Bei Versuchen im kleinen Maßstab werden diese Werkstoffe durch Fluorwasserstoff oder Gemische, die daneben noch weitere aggressive Bestandteile enthalten, nicht schwerwiegend geschädigt. Dagegen wird beim Arbeiten im technischen Maßstab, insbesondere beim kontinuierlichen Betrieb, eine wesentlich höhere Beständigkeit gefordert. Nach E. Rabald und H. Bretschneider (DECHEMA-Werkstofftabelle DWT 699, Nov. 1957) gelten für technische Zwecke Silber und Nickel sowie Nickellegierungen, insbesondere Monelmetall, das aus Nickel und Kupfer legiert ist, als die beständigsten Werkstoffe gegen die Korrosion durch Fluorwasserstoff unter verschiedensten Bedingungen. Auch in der Eu.Pat.Anm. 31 886 werden für die technische Durchführung der Isobuttersäuresynthese aus Propylen, Kohlenmonoxid und Wasser in Fluorwasserstoff als Koch'schem Katalysator Vorrichtungen aus Nickel oder Nickellegierungen empfohlen. Als Beispiele werden die unter dem Warenzeichen Monel, Inconel und Hastalloy gehandelten Nickellegierungen genannt, insbesondere eine Legierung aus 64 % Ni, 16 % Cr, 3 % Fe, 16 % Mo, 1 % Mn, 2 % Co (® Hastalloy C4).

In US-A 2 768 983 werden für die Umsetzung von Kohlenwasserstoffen mit wasserfreiem Fluorwasserstoff Apparaturen aus Aluminium vorgeschlagen. für die Durchführung der Koch'schen Synthese mittels Fluorwasserstoff eignen sich Aluminiumapparaturen nur dann, wenn sie für eine kurze Lebensdauer vorgesehen sind oder wenn infolge einer niedrigen Betriebstemperatur oder einer geringen Korrosivität des Gemisches nur ein langsamer Materialabtrag stattfindet.

Aus der US-A 3 993 475 ist eine Legierung aus 20-30 % Chrom, 26-40 % Nickel, geringen Anteilen Kohlenstoff, Mangan, Silicium, Wolfram und Kobalt und zum restlichen Teil aus Eisen bekannt, die sich durch hohe Kriechfestigkeit bei 1 100 bis 1 200 °C und durch hohe Beständigkeit gegen oxydative Korrosion auszeichnet. Über das Verhalten dieser Legierung gegenüber dem stark sauren, reduzierenden Medium der Koch'schen Synthese ist nichts bekannt.

### Aufgabe

Bei der Anwendung der genannten Metalle in der Praxis haben sich unerwartete Probleme ergeben. Die unter dem Handelsnamen Hastalloy C4 bekannt Legierung erwies sich zwar unter den Bedingungen der Koch'schen Carbonsäuresynthese als beständig, jedoch ist sie — vermutlich wegen des hohen Molybdängehaltes — so teuer, daß die Herstellung eines technischen Hochdruckreaktors aus diesem Metall zu wirtschaftlich kaum noch vertretbaren Apparatekosten führt.

Nickel selbst und zahlreiche Nickellegierungen, insbesondere das als HF-beständig geltende Monelmetall, erwiesen sich im Gegensatz zu den Erwartungen, die aus technischen Werkstofftabellen herleitbar waren, den äußerst aggressiven Gemischen der Koch'schen Carbonsäuresynthese bei Temperaturen über 80 °C oder über 100 °C nicht gewachsen. Es war ein Materialabtrag bis zu mehreren Millimetern pro Jahr festzustellen. Daher können die Erfahrungen über die Korrosionsbeständigkeit gegenüber Fluorwasserstoff oder wäßrige Fluorsäure nicht auf die Gemische der Koch'schen Synthese, die zusätzlich organische Carbonsäuren und Kohlenmonoxid enthalten können, übertragen werden. Es stellte sich die Aufgabe, ein Metall zu finden, das den gestellten Korrosionsbedingungen gewachsen ist und durch einen niedrigen Gehalt an hochwertigen Legierungsbestandteilen zu einem Preis erhältlich ist, der die Erstellung wirtschaftlich tragbarer Umsetzungs- und Aufarbeitungsvorrichtungen zuläßt. Die Aufgabe wurde erfindungsgemäß durch die Verwendung der Metallvorrichtung gemäß Hauptanspruch gelöst.

### Zusammensetzung der Metalle

Die erfindungsgemäß verwendeten Nickel-Chrom-Eisen-Legierungen enthalten

0 076 367

30-50 Gew.-% Ni
20-30 Gew.-% Cr
18-50 Gew.-% Fe.

Weitere Metalle können in untergeordneten Mengen enthalten sein, z. B. 3-7 Gew.-% Mo, 1,5 Gew.-% Mn, 2-5 Gew.-% Co, 1-1,5 Gew.-% W, etwa 2 Gew.-% Cu oder Nb. Der Anteil dieser Metalle liegt insgesamt unter 20 Gew.-% und für jedes Metall allein unter 10 Gew.-%. Bevorzugte Legierungen dieser Gruppe haben die Zusammensetzung :

30-45 Gew.-% Ni
20-25 Gew.-% Cr
30-50 Gew.-% Fe.

Sie sind wegen des zugunsten von Fe verminderten Ni- und Cr-Gehalts besonders preisgünstig, aber nicht weniger korrosionsbeständig als Legierungen mit höherem Ni- und Cr-Gehalt. Hinsichtlich des Preises und des Korrosionsverhaltens stellen die Legierungen des Typs

30-40 Gew.-% Ni
20-25 Gew.-% Cr
40-50 Gew.-% Fe

ein Optimum dar. Im allgemeinen sind keine weiteren Legierungsbestandteile darin enthalten.

Vorteilhafte Wirkungen

Die erhöhte Korrosionsbeständigkeit der gekennzeichneten Metalle läßt sich bei einer Korrosionsbehandlung über 500 Std. unter den Bedingungen der Koch'schen Synthese zeigen. Probestücke der untersuchten Metalle wurden einem Reaktionsgemisch der Koch'schen Synthese mit etwa 70 Gew.-% Fluorwasserstoff, 30 Gew.-% Isobuttersäure und untergeordneten Mengen weiterer Bestandteile bei 120 °C unter einem CO-Druck von 120 bar in der Weise ausgesetzt, daß sich jeweils ein Probestück in der Flüssigphase und in der Gasphase befand. Nach 500-stündiger Einwirkung wurde leicht entfernbarer Belag mit einer Messingbürste abgekratzt und der ermittelte Gewichtsverlust auf den Dickenabtrag pro Jahr umgerechnet.

| | Legierung (die Zahlen bedeuten Gew.-%) | | | | Abtrag in der Flüssigphase in mm/a |
|---|---|---|---|---|---|
| | Ni | Fe | Cr | andere | |
| A) | 46 | 19,5 | 22 | 6,5 Mo; 1,5 Mn; 2,5 Co; 1 W | 0,11 |
| B) | 46,5 | 19,5 | 22 | 7 Mo; 5 Co; 1,5 W; 1,9 Cu | 0,09 |
| C) | 40 | 33,3 | 21 | 3 Mo; 2 Cu | 0,06 - 0,003 |
| D) | 32 | 46,3 | 21 | – | - 0,084* |

Vergleich mit anderen HF-beständigen Legierungen :

| | | |
|---|---|---|
| F) | Reinnickel | 9,8 |
| G) | Monelmetall (65 % Ni, 34 % Cu, 1 % Mn) | 25 |

*) Die Legierung D ergab eine Gewichtszunahme (rechnerisch ein negativer Abtragswert) und hatte einen festen, glatten, emailartigen Überzug gebildet, der sich mit einer Messingbürste nicht abkratzen ließ.

Der Materialabtrag in der Gasphase war im allgemeinen noch geringer als in der Flüssigphase, bei Nickel mit 11,3 mm/a aber noch höher.

Die mit A bis D bezeichneten Metalle sind wesentlich billiger als andere Legierungen, die mittels eines hohen Molybdängehalts auf äußerste Korrosionsbeständigkeit eingestellt worden sind. In den

3

0 076 367

erfindungsgemäß eingesetzten Legierungen liegt der Molybdängehalt stets unter 10 Gew.-%.

Gewerbliche Anwendbarkeit

Die korrodierende Wirkung von Gemischen aus wesentlichen Mengen von Fluorwasserstoff neben organischen Carbonsäuren, Wasser und/oder Kohlenmonoxid tritt sowohl bei der Umsetzung entsprechender Gemische, inbesondere bei der Koch'schen Synthese, als auch bei der Aufarbeitung des Umsetzungsgemisches z. B. durch Destillation oder Extraktion auf. Zum Bereich der Umsetzungsvorrichtungen gehören nicht nur Reaktoren, sondern auch Rohrleitungen, Ventile, Pumpen und sonstige Vorrichtungen, durch die das Gemisch in den Reaktor hineingeführt oder aus ihm herausgeleitet werden. Zum Bereich der Aufarbeitungsvorrichtungen zählen z. B. Wärmetauscher, Destillations- und Extraktionssäulen, Lager- und Zwischenbehälter sowie die oben für den Umsetzungsbereich genannten Hilfsvorrichtungen. Alle diese Vorrichtungen werden hier insoweit als « Gefäße » bezeichnet, als die das korrodierende Gemisch in ihrem Innenraum enthalten können.

Die für die Berührung mit dem aggressiven Gemisch bestimmten Geräte werden erfindungsgemäß aus den gekennzeichneten Metallen gefertigt oder damit ausgekleidet und bei der Umsetzung und Aufarbeitung der Gemische verwendet. Aus Herstellungsgründen werden Gefäße bevorzugt, die gänzlich aus diesen Metallen bestehen. Für die Korrosionsbeständigkeit reicht es jedoch aus, wenn wenigstens die dem Innenraum zugewandte Schicht der Metallwandung aus den gekennzeichneten Metallen besteht. Die Mindestdicke dieser Schicht richtet sich nach dem Materialabtrag in der vorgesehenen Betriebsdauer. Wenn mit einem Materialabtrag von z. B. 0,01 bis 0,1 mm/a zu rechnen ist und die Vorrichtung mindestens 10 Jahre in Betrieb bleiben soll, sind Schichtdicken von mindestens 0,1 bis 1 mm vorzusehen. Um Korrosionsschäden sicher auszuschließen, werden Schichtdicken von 0,5 bis 5 mm bevorzugt. Ein solcher Schichtaufbau bietet sich vor allem für Druckgefäße mit einer Druckfestigkeit von z. B. 100 bar oder mehr an. Die äußere Schicht kann den weitaus überwiegenden Teil der Wandung bilden und aus nicht korrosionsbeständigem Stahl bestehen. Manchmal werden Reaktoren innen mit einer nichtmetallischen, meist keramischen Schicht ausgekleidet oder ausgemauert. Diese Auskleidungen sind gegen den korrodierenden Inhalt nicht dicht. Daher muß auch in diesen Fällen die dem Innenraum zugekehrte Seite der Metallwandung im Sinne der Erfindung aufgebaut sein.

Die Korrosionsbedingungen können sich an verschiedenen Stellen einer großtechnischen Anlage erheblich unterscheiden, so daß es sich aus Kostengründen empfiehlt, an weniger beanspruchten Stellen billigere Werkstoffe von entsprechend geringerer Korrosionsbeständigkeit einzusetzen. Unter Umständen genügt es, Gefäße aus den gekennzeichneten Metallen nur da einzusetzen, wo Temperaturen im Bereich von 80 bis 160°, insbesondere 100 bis 140 °C, auftreten. Auch die Zusammensetzung der Gemische kann an verschiedenen Stellen verschieden sein und unterschiedliche Korrosionsfestigkeiten erfordern. Das Wort « Gemisch » bezieht sich dabei nicht nur auf homogene flüssige oder gasförmige Phasen, sondern auch getrennte flussige oder gasförmige Phasen von gleicher oder verschiedener chemischer Zusammensetzung, sofern sie in einem Gefäß gleichzeitig vorliegen können.

Die zur Umsetzung eingesetzten Gemische enthalten oft keine Carbonsäuren, dafür aber manchmal Wasser, das in Anteilen über 5 %, bezogen auf das Gesamtgewicht des Gefäßinhalts, die Aggressivität spürbar erhöht. Organische Carbonsäuren in einer Konzentration von mehr als 10 Gew.-% wirken ebenfalls korrosionsfördernd.

Es wird angenommen, daß Kohlenmonoxid durch die Bildung von Carbonylen auf nickelhaltige Legierungen Korrodierend einwirkt. Seine zusätzliche korrodierende Wirkung macht sich bei CO-Partialdrucken über 10 bar bemerkbar. Das würde zwar die unbefriedigende Beständigkeit von Nickel und Monelmetall erklären, aber nicht die überraschende Beständigkeit der eisenhaltigen Legierungen, denn Eisen bildet selbst Carbonyle. Die höchste Korrosionsbelastung wirkt meistens auf den Umsetzungsreaktor selbst ein, weil dort alle Ausgangsstoffe und Endprodukte gleichzeitig auftreten und weil dort meistens besonders hohe Temperaturen und Drucke herrschen. Die erfindungsgemäße verwendeten Vorrichtungen dienen daher vorzugsweise als Umsetzungsgefäße.

Die Verwendung der Gefäße in kontinuierlich betriebenen Umsetzungs- und Aufarbeitungsanlagen bildet ein bevorzugtes Anwendungsgebiet der Erfindung. Die Koch'sche Synthese unter Einsatz eines Reaktionsgemisches, das wesentliche Mengen an propylen, Kohlenmonoxid, Fluorwasserstoff und gegebenenfalls Wasser oder einen niederen Alkanol enthält, ist das wichtigste Anwendungsgebiet.

**Patentansprüche**

1. Verwendung einer Umsetzungs- oder Aufarbeitungsvorrichtung aus Metall, wobei wenigstens die dem innenraum zugewandte Seite Metallwandung aus einen Nickel-Chrom-Eisen-Legierung mit

30 bis 50 Gew.-% Nickelgehalt
20 bis 30 Gew.-% Chromgehalt
18 bis 50 Gew.-% Eisengehalt

4

besteht, als Gefäß für die Umsetzung oder Aufarbeitung eines mindestens 30 Gew.-% Fluorwasserstoff neben organische Carbonsäuren und/oder Kohlenmonoxid und/oder Wasser enthaltenden Gemisches.

2. Verwendung einer Vorrichtung gemäß Anspruch 1, dadurch gekennzeichnet, daß wenigstens die innere Wandung aus einer Legierung mit

20 bis 25 Gew.-% Chromgehalt
30 bis 45 Gew.-% Nickelgehalt
30 bis 50 Gew.-% Eisengehalt
besteht.

3. Verwendung einer Vorrichtung gemäß Anspruch 2, dadurch gekennzeichnet, daß wenigstens die innere Wandung aus einer Legierung mit

20 bis 25 Gew.-% Chromgehalt
30 bis 40 Gew.-% Nickelgehalt
40 bis 50 Gew.-% Eisengehalt
besteht.

4. Verwendung einer Vorrichtung gemäß den Ansprüchen 1 bis 3, dadurch gekennzeichnet, daß sie als Druckgefäß mit einer Druckfestigkeit bis mindestens 100 bar ausgebildet ist.

5. Verwendung einer Vorrichtung gemäß den Ansprüchen 1 bis 4, dadurch gekennzeichnet, daß das umzusetzende oder aufzuarbeitende Gemisch über 5 Gew.-% Wasser enthält.

6. Verwendung einer Vorrichtung gemäß den Ansprüchen 1 bis 5, dadurch gekennzeichnet, daß sie bei der kontinuierlichen Umsetzung bzw. Aufarbeitung des in Anspruch 1 angegebenen Gemisches verwendet wird.

7. Verwendung einer Vorrichtung gemäß den Ansprüchen 1 bis 6, dadurch gekennzeichnet, daß sie bei Betriebstemperaturen von 80 bis 160°, vorzugsweise 100 bis 140°, verwendet wird.

8. Verfahren zur Umsetzung oder Aufarbeitung eines Koch'schen Reaktionsgemisches das wesentliche Mengen an Propylen, Kohlenmonoxid, Fluorwasserstoff und gegebenenfalls Wasser oder einen niederen Alkohol enthält, unter Druck- und Temperaturbedingungen, die für die Koch'sche Synthese üblich sind, dadurch gekennzeichnet, daß man die Umsetzung oder Aufarbeitung in einer nach den Ansprüchen 1 bis 4 zu verwendenden Vorrichtung durchführt.

**Claims**

1. Use of a reaction or working-up apparatus made of metal, in which at least the side of the metal wall facing the interior consists of a nickel-chromium-iron alloy containing

30 to 50 % by weight of nickel
20 to 30 % by weight of chromium
18 to 50 % by weight of iron,

as a vessel for the reaction or working up of a mixture containing at least 30 % by weight of hydrogen fluoride in addition to organic carboxylic acids and/or carbon monoxide and/or water.

2. Use of an apparatus as claimed in claim 1, characterised in that at least the inner wall consists of an alloy containing

20 to 25 % by weight of chromium
30 to 45 % weight of nickel
30 to 50 % by weight of iron.

3. Use of an apparatus as claimed in claim 2, characterised in that at least the inner wall consists of an alloy containing

20 to 25 % by weight of chromium
30 to 40 % by weight of nickel
40 to 50 % by weight of iron.

4. Use of an apparatus as claimed in claims 1 to 3, characterised in that it is constructed as a pressure vessel with a pressure resistance of at least 100 bar.

5. Use of an apparatus as claimed in claims 1 to 4, characterised in that the mixture which is to be reacted or worked up contains more than 5 % by weight of water.

6. Use of an apparatus as claimed in claims 1 to 5, characterised in that it is used in the continuous reaction or working up of the mixture specified in claim 1.

7. Use of an apparatus as claimed in claims 1 to 6, characterised in that it is used at operating temperatures of from 80 to 160°, preferably from 100 to 140°.

8. Process for reacting or working up a Koch reaction mixture which contains substantial amounts of propylene, carbone monoxide, hydrogen fluoride and optionally water or a lower alcohol, under the pressure and temperature conditions which are conventional for Koch synthesis, characterised in that the reaction or working up is carried out in an apparatus used in accordance with claims 1 to 4.


**Revendications**

1. Utilisation d'un dispositif de mise en réaction ou de traitement ultérieur en métal, dont au moins le côté de la paroi métallique faisant face à la cavité intérieure est fait d'un alliage de nickel-chrome-fer ayant

une teneur en nickel de 30 à 50 % en poids,
une teneur en chrome de 20 à 30 % en poids,
une teneur en fer de 18 à 50 % en poids,

comme récipient pour la mise en réaction ou le traitement ultérieur d'un mélange qui contient au moins 30 % en poids d'acide fluorhydrique, outre des acides carboxyliques organiques et/ou de l'oxyde de carbone et/ou de l'eau.

2. Utilisation d'un dispositif selon la revendication 1, caractérisé en ce qu'au moins la paroi intérieure est faite d'un alliage ayant

une teneur en chrome de 20 à 25 % en poids, ·
une teneur en nickel de 30 à 45 % en poids,
une teneur en fer de 30 à 50 % en poids.

3. Utilisation d'un dispositif selon la revendication 2, caractérisé en ce qu'au moins la paroi intérieure est faite d'un alliage ayant

une teneur en chrome de 20 à 25 % en poids,
une teneur en nickel de 30 à 40 % en poids,
une teneur en fer de 40 à 50 % en poids.

4. Utilisation d'un dispositif selon l'une quelconque des revendications 1 à 3, caractérisé en ce qu'il est réalisé sous forme de récipient de pression ayant une résistance à la pression qui atteint au moins 100 bars.

5. Utilisation d'un dispositif selon l'une quelconque des revendications 1 à 4, caractérisée en ce que le mélange devant être mis en réaction ou soumis au traitement ultérieur contient plus de 5 % en poids d'eau.

6. Utilisation d'un dispositif selon l'une quelconque des revendications 1 à 5, caractérisé en ce qu'il est utilisé dans la mise en réaction ou le traitement ultérieur en continu du mélange indiqué dans la revendication 1.

7. Utilisation d'un dispositif selon l'une quelconque des revendications 1 à 6, caractérisé en ce qu'il est utilisé à des températures de service de 80 à 160 °C, de préférence de 100 à 140 °C.

8. Procédé pour la mise en réaction ou le traitement ultérieur d'un mélange de réaction de Koch qui contient des quantités importantes de propylène, d'oxyde de carbone, d'acide fluorhydrique et éventuellement d'eau ou d'un alcanol inférieur, dans des conditions de pression et de températures qui sont habituelles pour la synthèse de Koch, caractérisé en ce qu'on mène la réaction ou le traitement ultérieur dans un dispositif à utiliser selon les revendications 1 à 4.